# DEMANDE DE BREVET EUROPEEN

(11) **EP 0 797 964 A1**
(43) Date de publication de la demande: **01.10.1997**
(21) Numéro de dépôt: 97420049.5
(22) Date de dépôt: 25.03.1997
(51) Int. Cl.: A61F 2/36, A61F 2/34

(54) **Ensemble d'éléments pour la constitution d'une prothése totale de la hanche**

(30) Priorité: 26.03.1996 FR 9604021
(71) Demandeur: Groupe Lepine, F-69003 Lyon (FR)
(72) Inventeur: Allieu, Yves, 34000 Montpellier (FR); Martin, Bruno, 30000 Nimes (FR); Aubin, Francis, 30000 Nimes (FR); ter Schiphorst, Patrick, 12000 LE Monastere (FR); Lamolinerie, Guy, 12100 Millau (FR); Trouillas, Joel, 30440 Sumene (FR)
(74) Mandataire: Guerre, Dominique

(57) **Abrégé**

Cet ensemble comprend une tige fémorale (2) et différents cols interchangeables (3) comprenant chacun une collerette de calage (20) munie de saillies radiales, destinée à être engagée dans une cavité d'assemblage (9).

Selon l'invention, la collerette (20) est dépourvue de saillies radiales et présente une forme sensiblement arrondie au niveau de sa partie (20a) se trouvant, après montage du col, sur le côté intérieur de la prothèse, et la paroi (8) de la tige délimitant la cavité d'assemblage (9) sur ce même côté intérieur de la prothèse présente une face interne (8a) de forme sensiblement arrondie, complémentaire de celle de ladite partie (20a) de la collerette.

Un élément cotyloïdien dont la coiffe comprend des saillies d'accrochage est également décrit.

## Description

La présente invention concerne un ensemble d'éléments pour la constitution d'une prothèse totale de la hanche.

Une telle prothèse comprend classiquement une tige médullaire fixée au fémur et un élément en forme de cupule fixé au cotyle. La tige fémorale est prolongée, à sa partie supérieure, par un col comportant une tête sphérique, et l'élément cotyloïdien délimite une cavité sphérique, qui reçoit la tête de la tige et permet le pivotement multidirectionnel de celle-ci.

Le col fait généralement corps avec la tige fémorale. Il existe toutefois des prothèses dans lesquelles ce col forme une pièce distincte de la tige, et peut être fixé sur cette tige par emmanchement. Ces prothèses comprennent plusieurs cols de différentes longueurs ou orientations pouvant être adaptés sur la tige en fonction des besoins.

Le document FR-A-2 693 367 décrit une tige fémorale à cols interchangeables. Chaque col comprend une collerette munie de saillies radiales régulièrement réparties sur sa périphérie, qui peut être engagée dans une cavité de forme complémentaire aménagée dans la partie supérieure de la tige. Les saillies de la collerette et les évidements complémentaires de la cavité constituent des moyens à indexation permettant la fixation du col sur la tige selon une pluralité de positions angulaires distinctes autour de l'axe d'assemblage. Lorsqu'un col coudé est utilisé, il est possible de régler l'orientation par rapport à la tige de la partie du col supportant la tête sphérique.

Une même tige peut ainsi être adaptée aux différentes morphologies d'articulation pouvant se rencontrer, au moyen d'un nombre de cols réduit.

Toutefois, il apparaît que les efforts verticaux appliqués sur la tête sphérique par le poids du patient génèrent des contraintes importantes et répétées au niveau de la périphérie de la cavité d'assemblage, d'autant plus que le col agit à la manière d'un levier. Ces contraintes s'exercent en particulier au niveau du ou des évidements aménagés pour recevoir les saillies de la collerette, qui sont situés sur le côté intérieur de la prothèse. Il peut en résulter une fatigue du matériau constituant la tige et une prise de jeu progressive du col, pouvant aboutir à la longue à un risque de rupture de la tige. Le ou les évidements précités viennent réduire l'épaisseur de la tige au niveau de ce côté intérieur de la prothèse, ce qui augmente encore ce risque de rupture.

La présente invention vise à remédier à cet inconvénient.

Par ailleurs, l'élément cotyloïdien d'une prothèse totale de hanche comprend une coiffe hémisphérique évidée, fixée au cotyle notamment par des vis, qui reçoit et retient un insert délimitant la cavité sphérique précitée.

Certains éléments cotyloïdiens connus sont faciles à implanter mais permettent un ancrage de résistance relativement incertaine dans le temps, et d'autres permettent un bon ancrage mais sont nettement plus difficiles à implanter.

L'invention vise également à remédier à cet inconvénient, en fournissant un implant cotyloïdien à la fois facile à implanter et permettant un ancrage résistant dans le temps.

L'ensemble d'éléments concerné comprend, de manière connue en soi, une tige fémorale, différents cols interchangeables pouvant être fixés sur cette tige, et au moins une tête sphérique d'articulation pouvant être montée sur ces cols, chaque col comprenant une collerette de calage munie de saillies radiales, destinée à être engagée dans une cavité de forme complémentaire aménagée dans la tige fémorale.

Selon l'invention, la collerette de chaque col est dépourvue de saillies radiales et présente une forme sensiblement arrondie au niveau de sa partie se trouvant, après montage du col, sur le côté intérieur de la prothèse, et la paroi de la tige délimitant la cavité d'assemblage sur ce même côté intérieur de la prothèse présente une face interne de forme sensiblement arrondie, complémentaire de celle de ladite partie de la collerette, cette partie de la collerette pouvant venir s'appliquer étroitement contre cette face arrondie lorsque le col est monté sur la tige.

Ainsi, la paroi délimitant la cavité d'assemblage sur le côté intérieur de la prothèse ne comprend pas d'évidement venant réduire son épaisseur dans sa zone subissant les contraintes les plus importantes, et la forme arrondie de la partie précitée de la collerette et de cette paroi permet une répartition homogène, sur une surface relativement importante, des contraintes transmises par le col à la tige.

Le risque de fatigue du matériau constituant la tige et de prise de jeu du col s'en trouve fortement réduit, voire même éliminé.

La prothèse selon l'invention ne comprend donc pas de col coudé orientable à volonté par pivotement autour de l'axe d'assemblage, mais une pluralité de cols de différentes conformations, dont chacun est assemblable selon une position unique. Cette position est déterminée par la forme de la collerette et de la cavité d'assemblage, qui constituent un détrompeur.

De préférence, l'ensemble d'éléments comprend au moins deux des cols suivants :
- un col rectiligne, ou plusieurs cols rectilignes de différentes longueurs ;
- un col "varisé", c'est-à-dire dont l'axe du pion supportant la tête sphérique est orienté, après montage, en direction du côté interne de la prothèse par rapport à l'axe d'assemblage ;
- un col "valgisé", c'est-à-dire dont l'axe du pion supportant la tête sphérique est orienté, après montage, en direction du côté externe de la prothèse par rapport à l'axe d'assemblage ;
- un col "antéversé" et un col "rétroversé", c'est-à-dire dont les axes des pions supportant la tête sphérique sont respectivement orientés, après montage, en direction du côté antérieur et du côté postérieur de la prothèse, par rapport à l'axe d'assemblage.

De préférence, pour ces différents cols, l'angle que forme l'axe du pion supportant la tête sphérique avec l'axe d'assemblage est de l'ordre de 15°. L'angle d'orientation latérale de ce même pion dans les cols "antéversé" ou "rétroversé" est de l'ordre de 60°.

La tige fémorale de la prothèse selon l'invention peut être soit fixée par scellement, soit ancrée par croissance osseuse au travers d'un revêtement poreux. Dans le premier cas, la tige présente une section transversale oblongue, sans arête vive, permettant d'éliminer toute contrainte localisée sur le ciment, et un bec en saillie sur le côté interne, à hauteur de la base du col, délimitant un rebord d'appui sur l'os. Dans le deuxième cas, la tige comprend, sur sa partie métaphysaire, une série d'évidements étagés qui délimitent des gradins successifs, dont certains sont orientés sensiblement perpendiculairement à l'axe longitudinal de la tige et descendent en direction de la partie distale de celle-ci, et dont les autres sont orientés sensiblement parallèlement à l'axe longitudinal de la tige et descendent en direction d'au moins un des côtés latéraux de celle-ci.

Ces gradins en renfoncement permettent de conserver un maximum d'os spongieux et assurent un bon calage de la tige dans le canal médullaire le temps que la croissance osseuse s'opère.

Les gradins sont avantageusement décalés les uns par rapport aux autres, au moins dans la zone centrale de la partie métaphysaire de la tige, en étant disposés à la manière "d'écailles de poisson". Cette disposition des gradins assure une parfaite prise d'appui de la tige contre l'os spongieux.

La coiffe de l'élément cotyloïdien comprend, quant à elle, deux saillies acérées en forme de bec, aménagées à distance l'une de l'autre dans la partie de la coiffe destinée à venir en regard de la paroi latérale du cotyle, et situées sensiblement à mi-hauteur de la paroi de la coiffe, et deux autres saillies délimitant des arêtes acérées orientées perpendiculairement au bord d'ouverture de la coiffe, aménagées à distance l'une de l'autre dans la partie de la coiffe destinée à venir en regard de la paroi supérieure du cotyle, des trous étant aménagés dans la partie de la coiffe destinée à venir en regard de la paroi latérale du cotyle pour permettre la mise en place de vis d'ancrage, et la face extérieure de la coiffe comprenant un revêtement poreux favorisant la croissance osseuse.

Les saillies précitées viennent s'insérer dans l'os spongieux lors de l'impaction de la coiffe. Les saillies en forme de bec assurent un maintien de la coiffe au contact de l'os et les saillies délimitant les arêtes acérées assurent une stabilisation en rotation de la coiffe. Cette dernière peut être mise en place dans la cavité cotyloïdienne par simple impaction et être maintenue en position d'implantation le temps que des vis d'ancrage soient fixées. La coiffe est ainsi particulièrement facile à implanter.

L'ancrage définitif est réalisé par croissance osseuse au travers du revêtement poreux.

La coiffe comprend avantageusement au moins deux fentes aménagées dans sa partie destinée à venir en regard de la paroi supérieure du cotyle, qui s'étendent depuis le bord délimitant l'ouverture de la coiffe jusqu'à la partie supérieure de celle-ci. Ces fentes permettent de conférer une certaine souplesse à la coiffe de manière à permettre de faciliter la mise en place de l'insert en polyéthylène dans la coiffe et la rétention de cet insert par simple encliquetage.

Pour sa bonne compréhension, l'invention est à nouveau décrite ci-dessous en référence au dessin schématique annexé représentant, à titre d'exemple non limitatif, une forme de réalisation préférée de plusieurs éléments appartenant à l'ensemble d'éléments qu'elle concerne.
La figure 1 est une vue en perspective d'une tige fémorale destinée à être fixée à l'os par scellement, et d'un col interchangeable adaptable sur cette tige ;
la figure 2 est une vue de cette tige selon l'axe d'assemblage de ce col ;
la figure 3 est une vue en coupe longitudinale de la partie supérieure de cette tige ;
la figure 4 est une vue de la tige en coupe selon la ligne IV-IV de la figure 1 ;
la figure 5 est une vue de profil d'une tige fémorale destinée à être fixée par croissance osseuse ;
la figure 6 est une vue de cette tige en coupe selon la ligne VI-VI de la figure 5 ;
la figure 7 est une vue de profil du col représenté à la figure 1 ;
la figure 8 en est une vue selon son axe d'assemblage à la tige ;
la figure 9 est une vue similaire à la figure 8 d'un autre col ;
la figure 10 est une vue en perspective d'un élément cotyloïdien, et
la figure 11 en est une vue en coupe selon la ligne XI-XI de la figure 10.

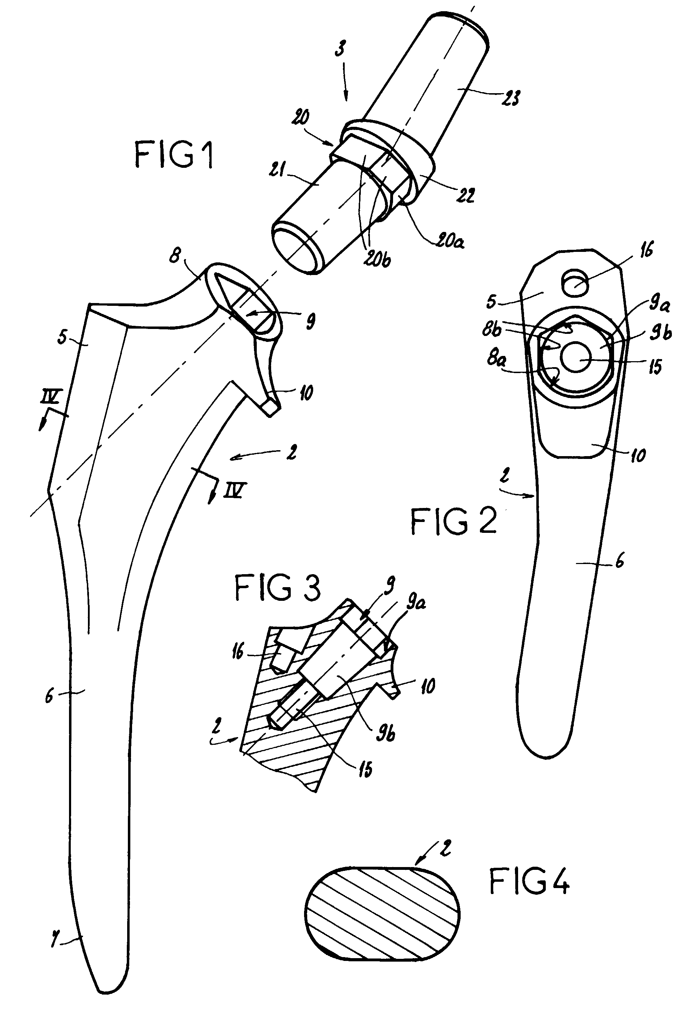

Les figures 1 à 4 et 7 à 9 représentent une tige médullaire 2 de prothèse de hanche destinée à être fixée à l'os par scellage au moyen d'un ciment synthétique, et deux cols interchangeables 3,4 pouvant être fixés sur l'extrémité supérieure de la tige 2.

La tige 2 présente une partie métaphysaire évasée 5 et une partie diaphysaire effilée 6, dont un pan 7 est coupé sur le côté externe de la prothèse. La tige 2 a une section transversale oblongue, sans arête vive sur l'ensemble de sa longueur, et comprend un bossage 8 à sa partie supérieure, délimitant une cavité 9 pour l'assemblage des cols 3,4, ainsi qu'un bec 10 sur son côté interne.

La cavité 9 présente, à partir de son ouverture, deux portions 9a,9b successives. Ainsi que le montre la figure 2, la paroi du bossage 8 délimitant la portion 9a présente une face interne 8a de forme arrondie, s'étendant sur pratiquement la moitié du bossage 8, du côté interne de la prothèse, et quatre facettes 8b disposées de manière hexagonale, s'étendant sur les côtés latéraux et sur le côté extérieur de la prothèse.

La portion 9b présente une forme conique à faible pente.

En outre, un alésage taraudé 15 débouche dans le fond de la partie 9b, et la tige 2 comprend un alésage taraudé 16 aménagé sur le côté externe de sa paroi supérieure. L'alésage 15 permet le vissage d'un instrument d'introduction de la tige 2 dans le canal osseux, et l'alésage 16 permet le vissage d'un instrument d'extraction de cette tige, pour le cas où une reprise ultérieure de la prothèse serait nécessaire.

Chaque col 3,4 comprend :
- une collerette de calage 20 présentant une forme complémentaire de celle de la portion 9a de la cavité 9, c'est-à-dire comprenant une partie arrondie 20a se trouvant sur le côté interne de la prothèse après montage, qui s'étend sur pratiquement la moitié de la périphérie de la collerette, et quatre facettes 20b disposées de manière hexagonale, s'étendant sur les côtés latéraux et le côté externe de la prothèse ;
- un pion conique d'assemblage 21, destiné à être emmanché en force dans la portion 9b de la cavité 9 ;
- une collerette d'appui 22, destinée à venir contre la face supérieure du bossage 8, et
- un pion conique 23, destiné à recevoir une tête sphérique d'articulation (non représentée), dont l'axe forme un angle d'environ 15° par rapport à l'axe du pion 21.

Ainsi que le montrent les figures 1 et 7 à 9, les pions 23 sont orientés différemment, d'un col à l'autre, par rapport aux pions 21 et aux parties arrondies 20a des collerettes 20.

Il apparaît à la figure 1 que le col 3 est "valgisé", c'est-à-dire qu'il comprend un pion 23 dont l'axe est orienté, après montage, en direction du côté externe de la prothèse par rapport à l'axe d'assemblage.

Le col 4 est un col "antéversé", c'est-à-dire qu'il comprend un pion 23 dont l'axe est orienté, après montage, en direction du côté antérieur de la prothèse par rapport à l'axe d'assemblage, selon un angle de l'ordre de 60°.

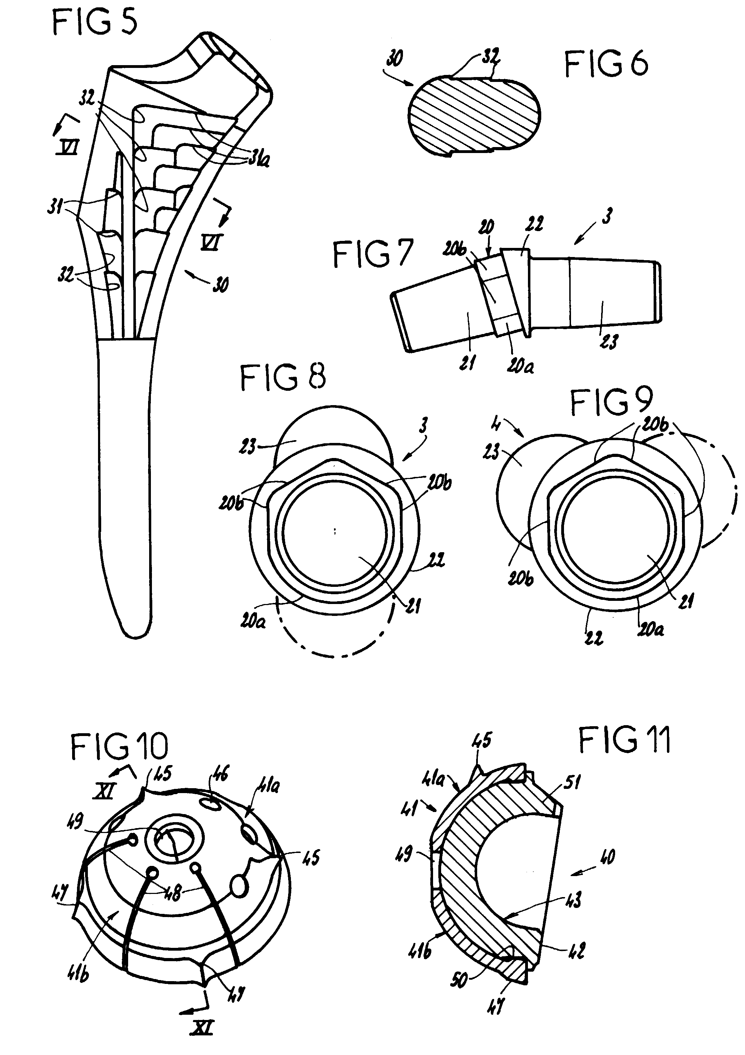

L'ensemble d'éléments selon l'invention comprend d'autres types de cols, non représentés. Notamment :
- un col "varisé", dont le pion supportant la tête sphérique (représenté en traits mixtes à la figure 8) a un axe orienté, après montage, en direction du côté interne de la prothèse par rapport à l'axe d'assemblage ;
- un col "rétroversé", dont le pion supportant la tête sphérique (représenté en traits mixtes à la figure 9) a un axe orienté, après montage, en direction du côté postérieur de la prothèse par rapport à l'axe d'assemblage, et
- un col rectiligne, ou plusieurs cols rectilignes de différentes longueurs.

En pratique, la tige 2 est introduite dans le canal fémoral, pour vérifier l'adaptation de ce canal à cette tige et pour déterminer, selon l'anatomie de l'articulation, quel est le col qui convient parmi ceux pouvant être adaptés sur la tige.

Le col choisi est ensuite assemblé à la tige 2 par engagement en force de son pion 21 dans la portion 9b de la cavité 9, jusqu'à venue de la collerette d'appui 22 au contact de la face supérieure du bossage 8. La collerette de calage 20 se trouve alors engagée dans la portion 9a de la cavité 9, avec venue de sa partie arrondie 20a et de ses facettes 20b à proximité immédiate de la face arrondie 8a et des facettes 8b correspondantes délimitées par le bossage 8.

La forme arrondie de cette partie 20a et de cette face 8a permet une répartition homogène, sur une surface relativement importante, des contraintes transmises par le col à la tige 2.

Le risque de fatigue du matériau constituant la tige 2 et de prise de jeu du col s'en trouve fortement réduit, voire même éliminé.

Ces partie 20a, face 8a et facettes 20b,8b constituent en outre un détrompeur permettant de déterminer une position unique d'assemblage pour le col choisi.

Après mise en place de la tête sphérique sur l'extrémité du col, l'ensemble est implanté dans l'os, jusqu'à venue de la saillie 10 en appui sur l'os. Le pan coupé 7 permet d'éviter tout contact de la tige 2 avec la corticale externe.

La figure 5 représente une tige fémorale 30 pouvant être utilisée en remplacement de la tige 2. Cette tige 30 est destinée à être ancrée par croissance osseuse au travers d'un revêtement poreux qu'elle comporte au niveau de sa partie métaphysaire.

Cette tige 30 est tout-à-fait similaire à la tige 2, sinon qu'elle est dépourvue de la saillie 10 d'appui sur l'os, et qu'elle comprend, sur sa partie métaphysaire, une série d'évidements étagés qui délimitent des gradins successifs 31,32. Les gradins 31 sont orientés sensiblement perpendiculairement à l'axe longitudinal de la tige 30 et descendent en direction de la partie distale de celle-ci. Les gradins 32 sont orientés sensiblement parallèlement à l'axe longitudinal de la tige 30 et descendent en direction des côtés interne et externe de la prothèse.

Les gradins 31,32 sont en outre décalés les uns par rapport aux autres, dans la zone centrale de la partie métaphysaire, en étant disposés à la manière "d'écailles de poisson".

Les figures 10 et 11 représentent un élément cotyloïdien 40 faisant également partie de l'ensemble d'éléments permettant de constituer la prothèse de hanche.

Cet élément 40 est constitué par une coiffe métallique 41 et un insert 42 en polyéthylène, inséré dans la coiffe 41. L'insert 42 délimite une cavité sphérique 43, qui reçoit la tête sphérique que comporte la tige 2 ou 30 et permet le pivotement multidirectionnel de celle-ci.

Ainsi que cela apparaît sur ces figures, la partie 41a de la coiffe 41 destinée à venir en regard de la paroi latérale du cotyle comprend deux saillies acérées 45 en forme de bec, aménagées à distance l'une de l'autre, sensiblement à mi-hauteur de la paroi de la coiffe 41, et des trous 46 permettant la mise en place de vis d'ancrage.

La partie 41b de la coiffe 41 destinée à venir en regard de la paroi supérieure du cotyle comprend, quant à elle, deux saillies 47 délimitant des arêtes acérées. Ces saillies 47 sont orientées perpendiculairement au bord d'ouverture de la coiffe 41 et sont aménagées à distance l'une de l'autre.

Cette même partie 41b comprend également trois fentes 48, s'étendant depuis le bord délimitant l'ouverture de la coiffe 41 jusqu'au niveau de la paroi supérieure de celle-ci.

La coiffe 41 comprend en outre une ouverture supérieure 49 de prise d'appui d'un instrument d'impaction, et est revêtue de revêtement poreux favorisant la croissance osseuse, tel que de l'hydroxyapatite de calcium.

Ainsi que le montre la figure 11, la coiffe 41 comprend un bourrelet périphérique 50 le long de son bord d'ouverture, sur lequel l'insert 42 vient s'engager par encliquetage, grâce à une gorge complémentaire qu'il comprend. Cet insert 42 peut comprendre un rebord antiluxation 51, comme montré à la figure 11, ou peut être dépourvu d'un tel rebord.

Les saillies 45,47 viennent s'insérer dans l'os spongieux lors de l'impaction de la coiffe 41, et assurent respectivement un maintien et une stabilisation en rotation de la coiffe 41 par rapport à l'os. Cette dernière peut être mise en place dans la cavité cotyloidienne par simple impaction et être maintenue en position d'implantation le temps que les vis d'ancrage soient mises en place.

Les fentes 48 confèrent une certaine souplesse à la coiffe 41, permettant de faciliter la mise en place et la rétention de l'insert 42 par simple encliquetage.

## Revendications

1. Ensemble d'éléments pour la constitution d'une prothèse totale de la hanche, comprenant une tige fémorale (2,30), différents cols interchangeables (3,4) pouvant être fixés sur cette tige, et au moins une tête sphérique d'articulation pouvant être montée sur ces cols, chaque col (3,4) comprenant une collerette de calage (20) munie de saillies radiales, destinée à être engagée dans une cavité (9) de forme complémentaire aménagée dans la tige fémorale (2,30), ensemble d'éléments caractérisé en ce que la collerette (20) de chaque col (3,4) est dépourvue de saillies radiales et présente une forme sensiblement arrondie au niveau de sa partie (20a) se trouvant, après montage du col, sur le côté intérieur de la prothèse, et la paroi (8) de la tige délimitant la cavité d'assemblage (9) sur ce même côté intérieur de la prothèse présente une face interne (8a) de forme sensiblement arrondie, complémentaire de celle de ladite partie (20a) de la collerette, cette partie (20a) de la collerette pouvant venir s'appliquer étroitement contre cette face arrondie (8a) lorsque le col est monté sur la tige (2,30).

2. Ensemble d'éléments selon la revendication 1, caractérisé en ce qu'il comprend au moins deux des cols suivants :
- un col rectiligne, ou plusieurs cols rectilignes de différentes longueurs ;
- un col "varisé", c'est-à-dire dont l'axe du pion supportant la tête sphérique est orienté, après montage, en direction du côté interne de la prothèse par rapport à l'axe d'assemblage ;
- un col (3) "valgisé", c'est-à-dire dont l'axe du pion (23) supportant la tête sphérique est orienté, après montage, en direction du côté externe de la prothèse par rapport à l'axe d'assemblage ;
- un col (4) "antéversé" et un col "rétroversé", c'est-à-dire dont les axes des pions (23) supportant la tête sphérique sont respectivement orientés, après montage, en direction du côté antérieur et du côté postérieur de la prothèse, par rapport à l'axe d'assemblage.

3. Ensemble d'éléments selon la revendication 2, caractérisé en ce que l'angle que forme l'axe du pion (23) supportant la tête sphérique avec l'axe d'assemblage est de l'ordre de 15°.

4. Ensemble d'éléments selon la revendication 2 ou la revendication 3, caractérisé en ce que l'angle d'orientation latérale du pion (23) supportant la tête sphérique dans les cols "antéversé" ou "rétroversé" est de l'ordre de 60°.

5. Ensemble d'éléments selon l'une des revendications 1 à 4, caractérisé en ce que la partie arrondie (20a) de la collerette de calage (20) s'étend sur pratiquement la moitié de la périphérie de la collerette, et en ce que cette collerette (20) comprend quatre facettes (20b) disposées de manière hexagonale, s'étendant sur les côtés latéraux et le côté externe de la prothèse.

6. Ensemble d'éléments selon l'une des revendications 1 à 5, caractérisé en ce que la tige fémorale (2), destinée à être fixée par scellement, présente une section transversale oblongue, sans arête vive et un bec (10) en saillie sur le côté interne, à hauteur de la base du col.

7. Ensemble d'éléments selon l'une des revendications 1 à 5, caractérisé en ce que la tige fémorale (30), destinée à être fixée par croissance osseuse au travers d'un revêtement poreux, comprend, sur sa partie métaphysaire, une série d'évidements étagés qui délimitent des gradins successifs (31,32), dont certains sont orientés sensiblement perpendiculairement à l'axe longitudinal de la tige (30) et descendent en direction de la partie distale de celle-ci, et dont les autres sont orientés sensiblement parallèlement à l'axe longitudinal de la tige (30) et descendent en direction d'au moins un des côtés latéraux de celle-ci.

8. Ensemble d'éléments selon la revendication 7, caractérisé en ce que les gradins (31,32) sont décalés les uns par rapport aux autres, au moins dans la zone centrale de la partie métaphysaire de la tige (30), en étant disposés à la manière "d'écailles de poisson".

9. Ensemble d'éléments selon l'une des revendications 1 à 8, comprenant un élément cotyloïdien (40) constitué par une coiffe (41) et par un insert (42), cet insert (42) délimitant une cavité sphérique (43) qui reçoit la tête sphérique que comporte la tige (2,30) et permet le pivotement multidirectionnel de celle-ci, élément cotyloïdien (40) caractérisé en ce que la coiffe (41) comprend deux saillies acérées (45) en forme de bec, aménagées à distance l'une de l'autre dans la partie (41a) de la coiffe (41) destinée à venir en regard de la paroi latérale du cotyle, et situées sensiblement à mi-hauteur de la paroi de la coiffe (41), et deux saillies (47) délimitant des arêtes acérées orientées perpendiculairement au bord d'ouverture de la coiffe (41), aménagées à distance l'une de l'autre dans la partie (41b) de la coiffe (41) destinée à venir en regard de la paroi supérieure du cotyle, des trous (46) étant aménagés dans la partie (41a) de la coiffe (41) destinée à venir en regard de la paroi latérale du cotyle pour permettre la mise en place de vis d'ancrage, et la face extérieure de la coiffe (41) comprenant un revêtement poreux favorisant la croissance osseuse.

10. Ensemble d'éléments selon la revendication 9, caractérisé en ce que la coiffe (41) comprend au moins deux fentes (48) aménagées dans sa partie (41b) destinée à venir en regard de la paroi supérieure du cotyle, qui s'étendent depuis le bord délimitant l'ouverture de la coiffe jusqu'à la partie supérieure de celle-ci.
